# EUROPEAN PATENT APPLICATION

(11) **EP 3 189 847 A1**
(43) Date of publication of application: **12.07.2017**
(21) Application number: 15837855.4
(22) Date of filing: 02.09.2015
(51) Int. Cl.: A61K 38/00, A61P 17/04, A61P 37/08, A61P 43/00, C07K 14/47, C12N 15/00

(54) **THERAPEUTIC OR PROPHYLACTIC AGENT FOR ITCHING SKIN DISEASES**

(30) Priority: 02.09.2014 JP 2014177778
(71) Applicant: Toray Industries, Inc., Tokyo 103-8666 (JP)
(72) Inventor: UMEHARA, Yoshie, Urayasu-shi Chiba 279-0021 (JP); KAMATA, Yayoi, Urayasu-shi Chiba 279-0021 (JP); TOMINAGA, Mitsutoshi, Urayasu-shi Chiba 279-0021 (JP); NIYONSABA, Francois, Tokyo 113-8421 (JP); TAKAMORI, Kenji, Urayasu-shi Chiba 279-0021 (JP)
(74) Representative: Kador & Partner
(86) International application number: PCT/JP2015/074906
(87) International publication number: WO 2016/035804

(57) **Abstract**

Disclosed is a novel means which enables treatment or prophylaxis of pruritic dermatoses such as atopic dermatitis. As a result of intensive study, the present inventors discovered that an antimicrobial peptide LL-37 has an action to promote expression of a nerve repulsion factor Sema3A. LL-37 is a peptide composed of the C-terminal 37 residues of human cathelicidin hCAP18. Use of this antimicrobial peptide enables normalization of expression of axon guidance molecules such as nerve repulsion factor in lesions of pruritic dermatoses including atopic dermatitis and xeroderma, and thus enables alleviation of pruritus and amelioration of skin lesions.

SEQUENCE LISTING

<110>  TORAY INDUSTRIES, INC.

<120>  Agent for treatment and/or prevention of pruritic dermatosis

<130>  PF545-PCT

<160>  19

<170>  PatentIn version 3.5

<210>  1
<211>  37
<212>  PRT
<213>  Homo sapiens

<400>  1

Leu Leu Gly Asp Phe Phe Arg Lys Ser Lys Glu Lys Ile Gly Lys Glu
1               5                   10                  15

Phe Lys Arg Ile Val Gln Arg Ile Lys Asp Phe Leu Arg Asn Leu Val
            20                  25                  30

Pro Arg Thr Glu Ser
        35

<210>  2
<211>  6
<212>  PRT
<213>  Artificial Sequence

<220>
<223>  PAR2 agonist peptide

<400>  2

Ser Leu Ile Gly Arg Leu
1               5

<210>  3
<211>  24
<212>  DNA
<213>  Artificial Sequence

<220>
<223>  Realtime PCR primer, Sema3A forward

<400>  3
acccaactat caatgggtgc ctta                                              24

<210>  4
<211>  24
<212>  DNA
<213>  Artificial Sequence

<220>

<223>  Realtime PCR primer, Sema3A reverse

<400>  4
aacactggat tgtacatggc tgga                                              24


<210>  5
<211>  19
<212>  DNA
<213>  Artificial Sequence

<220>
<223>  Realtime PCR primer, NGF forward

<400>  5
catgctggac ccaagctca                                                    19


<210>  6
<211>  20
<212>  DNA
<213>  Artificial Sequence

<220>
<223>  Realtime PCR primer, NGF reverse

<400>  6
cctgcaggga cattgctctc                                                   20


<210>  7
<211>  20
<212>  DNA
<213>  Artificial Sequence

<220>
<223>  Realtime PCR primer, KAL1 forward

<400>  7
caaccggatc agaggcatca                                                   20


<210>  8
<211>  20
<212>  DNA
<213>  Artificial Sequence

<220>
<223>  Realtime PCR primer, KAL1 reverse

<400>  8
ttggccgtat tggttggaca                                                   20


<210>  9
<211>  21
<212>  DNA
<213>  Artificial Sequence

<220>
<223>  Realtime PCR primer, Artemin forward

<400>  9

cccggaaagg tgcctagaag a                                                21

<210>  10
<211>  23
<212>  DNA
<213>  Artificial Sequence

<220>
<223>  Realtime PCR primer, Artemin reverse

<400>  10
atcagccatt gttgagctgt tcc                                              23

<210>  11
<211>  27
<212>  DNA
<213>  Artificial Sequence

<220>
<223>  Realtime PCR primer, hBD-1 forward

<400>  11
ttgtctgaga tggcctcagg tggtaac                                          27

<210>  12
<211>  26
<212>  DNA
<213>  Artificial Sequence

<220>
<223>  Realtime PCR primer, hBD-1 reverse

<400>  12
atacttcaaa agcaattttc ctttat                                           26

<210>  13
<211>  20
<212>  DNA
<213>  Artificial Sequence

<220>
<223>  Realtime PCR primer, hBD-2 forward

<400>  13
atcagccatg agggtcttgt                                                  20

<210>  14
<211>  20
<212>  DNA
<213>  Artificial Sequence

<220>
<223>  Realtime PCR primer, hBD-2 reverse

<400>  14
gagaccacag gtgccaattt                                                  20

<210>  15
<211>  23
<212>  DNA
<213>  Artificial Sequence

<220>
<223>  Realtime PCR primer, TSLP forward

<400>  15
tttatacctg ttggccctgc cta                                               23


<210>  16
<211>  20
<212>  DNA
<213>  Artificial Sequence

<220>
<223>  Realtime PCR primer, TSLP reverse

<400>  16
cttgcagcgg gaattcaaga                                                   20


<210>  17
<211>  25
<212>  DNA
<213>  Artificial Sequence

<220>
<223>  Realtime PCR primer, RPS18 forward

<400>  17
tttgcgagta ctcaacacca acatc                                             25


<210>  18
<211>  21
<212>  DNA
<213>  Artificial Sequence

<220>
<223>  Realtime PCR primer, RPS18 reverse

<400>  18
gagcatatct tcggcccaca c                                                 21


<210>  19
<211>  34
<212>  PRT
<213>  Mus musculus

<400>  19

Gly Leu Leu Arg Lys Gly Gly Glu Lys Ile Gly Glu Lys Leu Lys Lys
1               5                   10                  15


Ile Gly Gln Lys Ile Lys Asn Phe Phe Gln Lys Leu Val Pro Gln Pro
            20                  25                  30

Glu Gln

## Description

### TECHNICAL FIELD

The present invention relates to a therapeutic or prophylactic agent for pruritic dermatosis.

### BACKGROUND ART

A normal epidermal barrier plays roles in protecting the body against various stimulations from the outside, and in retaining an appropriate amount of moisture in the body. The epidermis is a physical barrier, and, moreover, the epidermis itself has a unique mechanism for recognition and elimination of pathogens, forming a barrier by natural immunity without involvement of lymphocytes.

In the mechanism for elimination of pathogens, epidermal keratinocytes produce human β-defencin (hBD) and cathelicidin as antimicrobial peptides (Non-patent Documents 1 and 2). These peptides have strong positive charges, and are inserted into the negatively charged cell membrane of bacteria to form pores, resulting in exertion of their bactericidal action (Non-patent Document 3). As human cathelicidin, LL-37 is well known. LL-37 is an antimicrobial peptide consisting of the C-terminal 37 amino acid residues of the 18 kDa inactive protein hCAP18, which peptide is released from hCAP18 through its degradation by serine protease kallikrein. In normal human epidermis, hBD-1, which has relatively weak antimicrobial activity, shows constant expression, while other antimicrobial peptides are induced in an emergency such as bacterial infection or inflammation caused by bacterial contact, differentiation, cytokine stimulation, or the like (Non-patent Document 4). Antimicrobial peptides are used in, for example, techniques developed for inhibiting the growth or metastasis of cancer cells such as breast cancer cells (Patent Document 1).

Antimicrobial peptides produced by epidermal keratinocytes in the skin increase in psoriatic lesions, and this has been thought to be one of the causes of less frequency of skin infections in psoriasis. It is reported that, in lesions of atopic dermatitis, formation of the bacterial flora of *Staphylococcus aureus*, which is not found in healthy individuals, is found, and that expression of antimicrobial peptides occurs only at lower levels compared to psoriatic lesions irrespective of the presence of inflammation in the epidermis (Non-patent Document 5). This abnormal expression of antimicrobial peptides in the epidermis of patients with atopic dermatitis has been considered to be one of the causes of extremely high frequencies of the growth of, and severe infection with, *Staphylococcus aureus.* Toxins and proteases produced by *Staphylococcus aureus* have been considered to contribute to chronicity and severity of atopic dermatitis (Non-patent Document 6).

In a preceding study by the group of the present inventors, increased expression of nerve growth factors (nerve growth factor [NGF], amphiregulin) and decreased expression of nerve repulsion factors (semaphorin 3A (Sema3A), anosmin-1), which cause retraction of nerve fibers, were found in the skin of patients with atopic dermatitis or xeroderma in whom the function of the skin barrier was disrupted, and the skin of their model mice. The study reported that this causes invasion of a number of sensory nerve fibers, which normally show convergence in the epidermis-dermis boundary, into the epidermis, and their hyperplasia therein (Non-patent Document 7). In a model animal for atopic dermatitis, an ointment containing a recombinant Sema3A suppressed scratching behavior, and ameliorated dermatitis (Non-patent Document 8). The above results strongly suggest that nerve repulsion factors can be therapeutic targets for intractable pruritus accompanied by invasion of nerve repulsion factors into the epidermis.

### PRIOR ART DOCUMENTS

### [Patent Documents]

Patent Document 1: JP 2009-541287 A
Patent Document 2: JP 2013-521300 A

### [Non-patent Documents]

Non-patent Document 1: Scott et al., Crit Rev Immunol, 2000: 20: 407-431
Non-patent Document 2: Weinberg et al., Crit Rev Oral Biol Med, 1998: 9: 399-414
Non-patent Document 3: Ganz et al., Nat Rev Immunol, 2003: 3, 710-720
Non-patent Document 4: Midorikawa et al., Infect Immun. 2003, 71(7): 3730-3739
Non-patent Document 5: Harder et al, J Invest Dermatol, 2010: 130, 1355-1364
Non-patent Document 6: Cork et al, J Invest Dermatol, 2009: 129, 1892-1908
Non-patent Document 7: Tominaga et al., Exp Rev Dermatol, 2010, 5, 197-212
Non-patent Document 8: Negi et al., J Dermatol Sci, 2012, 66: 37-43

### SUMMARY OF THE INVENTION

### PROBLEMS TO BE SOLVED BY THE INVENTION

Patent Documents 1 and 2, and Non-patent Documents 1 to 8 do not report association between expression of Sema3A and antimicrobial peptides in lesions of atopic dermatitis at all. Patent Document 2 discloses a technique in which a compound that isolates antimicrobial peptides such as LL-37 is used for treatment of skin diseases and skin disorders, but this is a mere technique that uses as an active ingredient a substance that sequesters LL-37, and the technique does not use an antimicrobial peptide itself such as LL-37 as a therapeutic agent for skin diseases.

An object of the present invention is to provide novel means which enables treatment or prevention of pruritic dermatoses such as atopic dermatitis.

### MEANS FOR SOLVING THE PROBLEMS

As a result of intensive study, the present inventors discovered that the antimicrobial peptide LL-37 has an action to promote expression of nerve repulsion factors such as Sema3A, and that use of the antimicrobial peptide enables normalization of expression of axon guidance molecules such as nerve repulsion factors in lesions of pruritic dermatoses including atopic dermatitis, to reduce pruritus and ameliorate skin lesions, thereby completing the present invention.

That is, the present invention provides a therapeutic or prophylactic agent for pruritic dermatosis comprising as an active ingredient a polypeptide of the following (a) or (b).
(a) A polypeptide with the amino acid sequence of SEQ ID NO:1;
(b) A polypeptide with the same amino acid sequence as the amino acid sequence of SEQ ID NO:1 except that one or several amino acids are substituted, deleted, inserted, and/or added, the polypeptide having therapeutic and/or prophylactic activity for pruritic dermatosis.

The present invention also provides an agent for promoting expression of semaphorin 3A, comprising as an active ingredient a polypeptide of the following (a) or (b').
(a) A polypeptide with the amino acid sequence of SEQ ID NO:1.
(b') A polypeptide with the same amino acid sequence as the amino acid sequence of SEQ ID NO:1 except that one or several amino acids are substituted, deleted, inserted, and/or added, the polypeptide having an activity to promote expression of Sema3A.

The present invention also provides a method of treating or preventing pruritic dermatosis, the method comprising administering an effective amount of a polypeptide of the above (a) or (b) to a subject in need thereof. The present invention also provides a method of promoting expression of Sema3A, the method comprising administering an effective amount of a polypeptide of the above (a) or (b') to a subject in need thereof.

### EFFECT OF THE INVENTION

By the present invention, a novel therapeutic or prophylactic agent for pruritic dermatoses, which agent targets axon guidance molecules, was provided. Local administration of the above-described polypeptide to lesions of pruritic dermatoses including atopic dermatitis enables normalization of expression of axon guidance molecules, and thus enables alleviation of pruritus and amelioration of skin lesions. Conventionally, standard therapeutic agents for pruritic dermatoses have been steroid drugs and antihistamines. Use of these agents is symptomatic treatment since they are agents for amelioration of symptoms by suppressing various pruritus/inflammation-causing substances that are released as a result of allergic reaction. Such agents are incapable of radical amelioration of a state where invasion of a number of sensory nerve fibers into the epidermis has occurred to cause hypersensitivity to pruritus. In contrast, the agent of the present invention, which targets axon guidance molecules, enables radical treatment of pruritus. Since the agent of the present invention has a mechanism different from those of conventional standard therapeutic agents, the agent of the present invention can be expected to have a therapeutic effect on refractory atopic dermatitis against which steroid drugs and antihistamines have become ineffective.

### BRIEF DESCRIPTION OF THE DRAWINGS

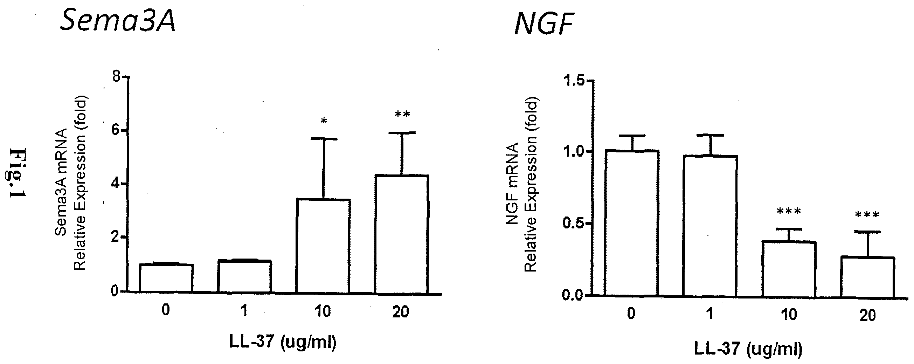

Fig. 1 shows the result of mRNA expression analysis of axon guidance molecules Sema3A and NGF in normal human epidermal keratinocytes (NHEKs) as observed 48 hours after stimulation with LL-37. It was confirmed that expression of the nerve repulsion factor Sema3A was promoted, and that expression of the nerve growth factor NGF was suppressed. *P<0.05, **P<0.01, ***P<0.001, significant differences from control (in which LL-37 was not added).

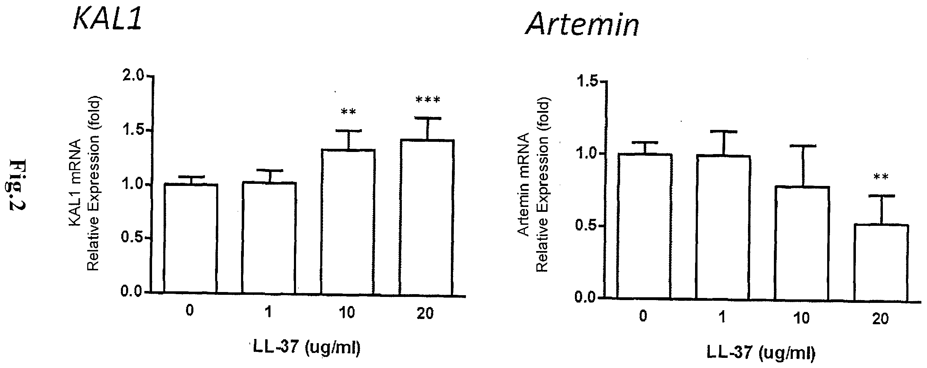

Fig. 2 shows the result of mRNA expression analysis of axon guidance molecules KAL1 and Artemin in NHEKs as observed 48 hours after stimulation with LL-37. It was confirmed that expression of the nerve repulsion factor KAL1 was promoted, and that expression of the nerve growth factor Artemin was suppressed. **P<0.01, ***P<0.001, significant differences from control (in which LL-37 was not added).

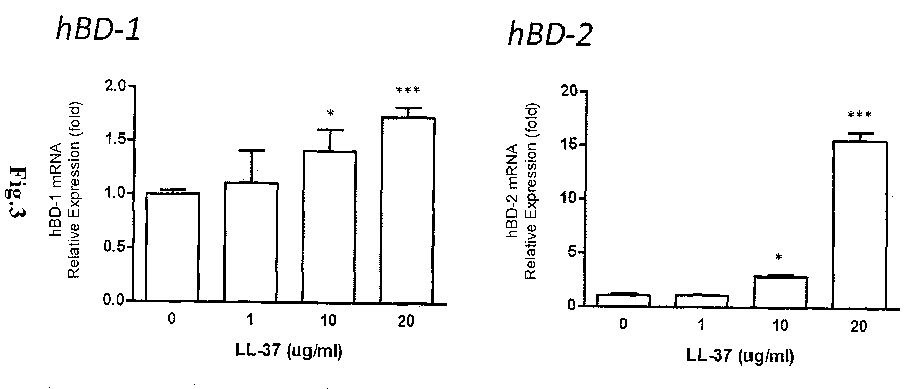

Fig. 3 shows the result of mRNA expression analysis of antimicrobial peptides hBD-1 and hBD-2 in NHEKs as observed 48 hours after stimulation with LL-37. Promotion of expression of both mRNAs was confirmed. *P<0.05, ***P<0.001, significant differences from control (in which LL-37 was not added).

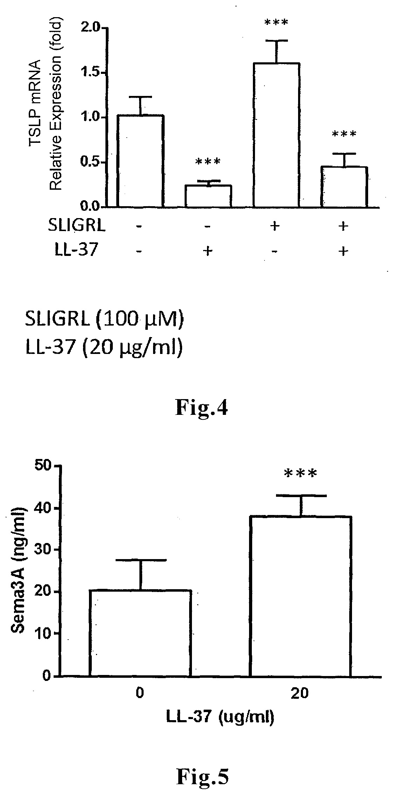

Fig. 4 shows the result of mRNA expression analysis of TSLP in NHEKs as observed 48 hours after stimulation with LL-37. Suppression of expression of TSLP was confirmed. It was further confirmed that suppression of mRNA expression of TSLP occurred by addition of LL-37 also when expression of TSLP was induced by addition of SLIGRL to NHEKs. ***P<0.001, significant differences from control (in which LL-37 was not added).
Fig. 5 shows the result of protein xpression analysis of an axon guidance molecule Sema3A in NHEKs as observed 48 hours after stimulation with LL-37. It was confirmed that the expression level of the nerve repulsion factor Sema3A increased. ***P<0.001, a significant difference from control (in which LL-37 was not added).

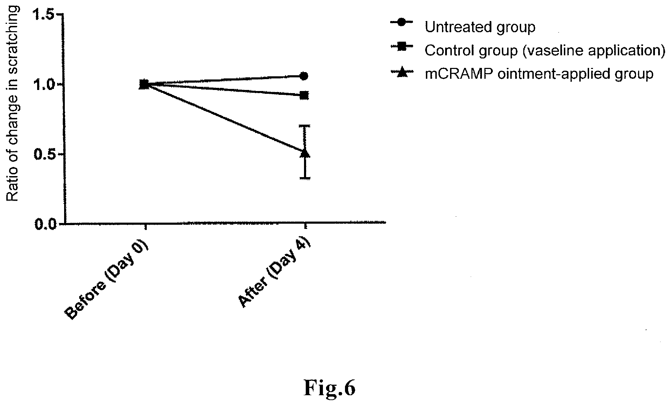

Fig. 6 shows the result of comparison of the ratio of change in the frequency of scratching behavior observed before and after application of 100 mg/mouse of mCRAMP ointment or vaseline twice a day for 4 days to Dfb-NC/Nga mice that developed atopic dermatitis. In the mCRAMP ointment-applied group (n=3), the frequency of scratching behavior was evidently lower than those in the untreated group (n=2) and the control group (n=2).

### MODE FOR CARRYING OUT THE INVENTION

The agent of the present invention is a therapeutic or prophylactic agent for pruritic dermatosis, comprising as an active ingredient a polypeptide described below.
(a) A polypeptide with the amino acid sequence of SEQ ID NO:1.
(b) A polypeptide with the same amino acid sequence as the amino acid sequence of SEQ ID NO:1 except that one or several amino acids are substituted, deleted, inserted, and/or added, the polypeptide having therapeutic and/or prophylactic activity for pruritic dermatosis.

SEQ ID NO:1 shows the amino acid sequence of the human cathelicidin LL-37. The present inventors revealed that LL-37 has actions to suppress expression of nerve growth factors and to promote expression of nerve repulsion factors in epidermal keratinocytes. It was also revealed that LL-37 is capable of suppressing expression of a cytokine TSLP in epidermal keratinocytes. It is known that, among axon guidance molecules, nerve growth factors such as NGF and amphiregulin show increased expression, while nerve repulsion factors such as Sema3A and anosmin-1 show decreased expression, in lesions of pruritic dermatoses such as atopic dermatitis and xeroderma (Non-patent Document 7). It is also reported that high expression of a cytokine TSLP is found in lesions of atopic dermatitis (Soumelis et al, Nat Immunol, 2002: 3, 673-680). Further, TSLP has recently been reported to induce not only inflammation, but also pruritus (Wilson et al, Cell, 2013: 155, 285-295). It is also known that application of a recombinant Sema3A to lesions allows alleviation of pruritus and amelioration of dermatitis (Non-patent Document 8). By application of LL-37 to lesions of skin diseases, expression of axon guidance molecules and TSLP can be normalized, and pathological conditions of pruritic dermatoses such as atopic dermatitis can be effectively ameliorated.

It is widely known to those skilled in the art that, in general, a protein may retain almost the same function as the original protein even if a small number of amino acid residues in the amino acid sequence of the protein are substituted, deleted, and/or inserted. Thus, polypeptides having the same amino acid sequence as the amino acid sequence of SEQ ID NO:1 except that one or several (for example, 1 to 9, 1 to 8, 1 to 7, 1 to 6, 1 to 5, 1 to 4, 1 to 3, 1 to 2, or 1) amino acids are substituted, deleted, inserted, and/or added, as defined in (b) above, can also be used for treatment or prevention of pruritic dermatoses similarly to the polypeptide (a) (a polypeptide having the same sequence as the sequence of LL-37) as long as they have therapeutic and/or prophylactic activity for pruritic dermatoses. The polypeptide (b) may have a sequence identity of, for example, not less than 80%, not less than 90%, or not less than 95% to SEQ ID NO:1.

As used herein, "sequence identity" means a value expressed in percentage which is calculated by aligning two amino acid sequences to be compared such that as many amino acid residues as possible are matched and then dividing the number of the matched amino acid residues by the number of the total amino acid residues. When the above-mentioned alignment is carried out, a gap(s) is/are appropriately inserted into one or both of the two sequences to be compared as required. Such alignment of sequences can be carried out using a well-known program such as BLAST, FASTA, or CLUSTAL W. In cases where a gap(s) is/are inserted, the above-mentioned number of the total amino acid residues is calculated by counting one gap as one amino acid residue. When the thus counted numbers of the total amino acid residues are different between the two sequences to be compared, the sequence identity (%) is calculated by dividing the number of the matched amino acid residues by the number of the total amino acid residues of the longer sequence. The 20 types of amino acids constituting naturally-occurring proteins can be classified into groups according to the similar property, for example, into the following groups: neutral amino acids with side chains having low polarity (Gly, Ile, Val, Leu, Ala, Met, Pro); neutral amino acids having hydrophilic side chains (Asn, Gln, Thr, Ser, Tyr, Cys); acidic amino acids (Asp, Glu); basic amino acids (Arg, Lys, His); and aromatic amino acids (Phe, Tyr, Trp). It is known that, in most cases, substitution of an amino acid(s) within the same group does not change the properties of a polypeptide. Therefore, when an amino acid residue in the polypeptide (a) is substituted, the therapeutic or prophylactic activity for pruritic dermatoses of the polypeptide to be used as the active ingredient is highly likely to be maintained by substitution within the same group.

The treatment of pruritic dermatoses includes amelioration of various skin lesions such as amelioration of pruritus and amelioration of inflammatory skin lesions. The prevention of pruritic dermatoses includes prevention of occurrence of skin lesions by amelioration of pruritus. The agent of the present invention may also be administered prophylactically before occurrence of pruritus to sites where pruritus has repeatedly occurred.

"Therapeutic or prophylactic activity for pruritic dermatoses" means activity to ameliorate symptoms in skin lesions of pruritic dermatoses, or to prevent exacerbation of the symptoms. The fact that antimicrobial peptides such as cathelicidin are effective for suppressing dermal pruritus is as shown below concretely in the following Examples. This effect is derived from the action of the antimicrobial peptides such as cathelicidin to normalize expression of any one or more of axon guidance molecules and the cytokine TSLP in the lesions, that is, any one or more actions to promote expression of nerve repulsion factors such as Sema3A and KAL1, to suppress expression of nerve growth factors such as NGF and Artemin, and to suppress expression of TSLP. Thus, whether or not an arbitrary polypeptide whose amino acid sequence is different from SEQ ID NO:1 in a small number of amino acids has a therapeutic or prophylactic activity for pruritic dermatoses can be evaluated by investigating, for example, whether or not the arbitrary polypeptide shows any one or more of actions such as promotion of expression or production of the above-described nerve repulsion factors, suppression of expression or production of the above-described nerve growth factors, and suppression of expression of TSLP, in epidermis-derived cultured cells such as human epidermal keratinocytes (preferably human epidermis-derived cultured cells). More simply, the therapeutic or prophylactic activity can be evaluated based on whether or not the polypeptide has an action to promote expression or production of Sema3A in epidermis-derived cells.

Since model animals showing symptoms of pruritic dermatoses are known, whether or not an arbitrary polypeptide has a therapeutic or prophylactic activity for pruritic dermatoses can also be evaluated by carrying out an experiment by administration to such model animals.

For example, as a model animal that develops atopic dermatitis, which is one of pruritic dermatoses, the NC/Nga mouse is known. The NC/Nga mouse is an animal model of atopic dermatitis that is widely used in the art. When it is kept under normal conditions, it develops dermatitis similar to atopic dermatitis on the face, ears, upper back, and the like from about 7 weeks old.

Another specific example of model animals that can be used for evaluation of the above activities is dry-skin model mice. It is known that dry skin is found in diseases such as atopic dermatitis, xeroderma, renal failure, and cholestatic liver diseases. A dry-skin model mouse can be prepared by, for example, shaving the hair on the back of an ICR mouse, and then applying cotton impregnated with an acetone solution to the skin for 5 minutes. A dry-skin model mouse can also be prepared by applying cotton impregnated with an acetone solution to the skin of a Hos:HR-1 hairless mouse for 5 minutes. In the dry-skin models, invasion of nerve fibers into the epidermis is observed from 24 hours after the acetone treatment, and abnormal expression of axon guidance molecules (increased expression of nerve growth factors NGF and amphiregulin, and decreased expression of a nerve repulsion factor Sema3A) is found similarly to atopic dermatitis model mice (Kamo et al., J Dermatol Sci 62 91-97, 2011).

The evaluation of the therapeutic or prophylactic activity for pruritic dermatoses using a model animal that develops atopic dermatitis can be carried out by, for example, administering the polypeptide to the model animal by application to the skin lesion, and evaluating whether the symptoms in the lesion are ameliorated or not. Whether the symptoms in the skin lesion are ameliorated or not can be evaluated by using a known index such as the clinical skin score (Leung, Clin Exp Immunol 107 (Suppl. 1), 25-30, 1997; Suto et al., Int Arch Allergy Immunol 120 (Suppl. 1), 70-75, 1999). For example, in cases where the mean score value in a disease-developing population to which the polypeptide was administered for a predetermined period was significantly lower than the mean score value in an untreated disease-developing population, or, for example, in cases where the mean score value in the polypeptide administration group is not more than about two thirds, preferably not more than about half, of the mean score value in the untreated population, the polypeptide can be determined as having a therapeutic or prophylactic activity for pruritic dermatoses.

The evaluation of the therapeutic or prophylactic activity for pruritic dermatoses using a model animal of atopic dermatitis or a dry-skin model animal can be carried out as follows. For example, in cases where the number of nerve fibers invading into the epidermis (it is noted here that the above-described disease models show increased numbers of nerve fibers invading into the epidermis) and/or abnormal expression of axon guidance molecules (increased expression of nerve growth factors NGF and amphiregulin, and decreased expression of a nerve repulsion factor Sema3A) is/are normalized compared to those in an untreated disease-developing population, the polypeptide can be determined as having a therapeutic or prophylactic activity for pruritic dermatoses.

Specific examples of "pruritic dermatoses" include, but are not limited to, atopic dermatitis, xeroderma, prurigo nodularis, chronic renal failure, cholestatic liver cirrhosis, primary biliary cirrhosis, dialysis pruritus, and visceral malignant tumors. The agent of the present invention can be especially preferably used for atopic dermatitis. The agent of the present invention can also be used for treatment or prevention of skin pruritus associated with an infection since the antimicrobial activity of the polypeptide also exerts a preferred effect on pathogens. However, the pruritic dermatosis to which the present invention is applied may be a disease other than skin pruritus associated with an infection. The pruritic dermatosis to which the present invention is applied may be a disease other than psoriasis.

"Polypeptide" in the present invention means a molecule formed by linking of a plurality (two or more) of amino acids through a peptide bond(s), and includes high-molecular-weight molecules constituted by large numbers of amino acids, as well as low-molecular-weight molecules (oligopeptides) having small numbers of amino acids, and proteins.

Generally in the field of pharmaceuticals composed of a polypeptide, in order to increase the *in vivo* stability of the polypeptide, techniques in which a sugar chain(s) and/or a polyethylene glycol (PEG) chain(s) is/are added to the polypeptide, or in which a D-amino acid(s) is/are used as at least part of the amino acids constituting the polypeptide, and the likeare widely known and used. The addition of a sugar chain(s) and/or a PEG chain(s), or the use of a D-amino acid(s) as at least part of the amino acids constituting a polypeptide makes the polypeptide more unlikely to be decomposed by peptidases in the body, and in turn, makes the *in vivo* half-life of the polypeptide longer. The polypeptide of the present invention may be a polypeptide that has been subjected to these known modifications for *in vivo* stability as long as the resulting polypeptide has a therapeutic or prophylactic activity for pruritic dermatoses. The term "polypeptide" as used in the present description and claims also includes those modified for *in vivo* stability unless the context clearly dictates otherwise.

Addition of a sugar chain to a polypeptide is well known, and described in, for example, Sato M, Furuike T, Sadamoto R, Fujitani N, Nakahara T, Niikura K, Monde K, Kondo H, Nishimura S., "Glycoinsulins: dendritic sialyloligosaccharide-displaying insulins showing a prolonged blood-sugar-lowering activity.", J Am Chem Soc. 2004 Nov 3, 126(43): 14013-22; and Sato M, Sadamoto R, Niikura K, Monde K, Kondo H, Nishimura S, "Site-specific introduction of sialic acid into insulin.", Angew Chem Int Ed Engl. 2004 Mar 12, 43(12):1516-20. Although a sugar chain can be bound to the N-terminus, the C-terminus, or any amino acids between them, it is preferred that a sugar chain be bound to the N-terminus or the C-terminus so that the activity of the polypeptide is not inhibited. The number of the sugar chains added is preferably one or two, preferably one. The sugar chain is preferably from mono- to tetra-saccharide, more preferably a disaccharide or trisaccharide. The sugar chain(s) can be bound directly to a free amino group(s) or a carboxyl group(s) on the polypeptide, or can be bound thereto through a spacer structure(s) such as a methylene chain whose number of carbon atoms is about 1 to 10.

Addition of a PEG chain to a polypeptide is also well known, and described in, for example, Ulbricht K, Bucha E, Poschel KA, Stein G, Wolf G, Nowak G., "The use of PEG-Hirudin in chronic hemodialysis monitored by the Ecarin Clotting Time: influence on clotting of the extracorporeal system and hemostatic parameters.", Clin Nephrol. 2006 Mar, 65(3): 180-90; and Dharap SS, Wang Y, Chandna P, Khandare JJ, Qiu B, Gunaseelan S, Sinko PJ, Stein S, Farmanfarmaian A, Minko T., "Tumor-specific targeting of an anticancer drug delivery system by LHRH peptide.", Proc Natl Acad Sci USA. 2005 Sep 6, 102(36): 12962-7. A PEG chain can be bound to the N-terminus, the C-terminus, or any amino acids between them. Usually, one or two PEG chains are bound to a free amino group(s) and/or carboxyl group(s) on the polypeptide. The molecular weight of the PEG chain is not particularly limited, and usually about 3000 to 7000, preferably about 5000.

Methods for preparing a polypeptide in which at least part of the amino acids constituting it are D-amino acids are also well known, and described in, for example, Brenneman DE, Spong CY, Hauser JM, Abebe D, Pinhasov A, Golian T, Gozes I., "Protective peptides that are orally active and mechanistically nonchiral.", J Pharmacol Exp Ther. 2004 Jun, 309(3): 1190-7; and Wilkemeyer MF, Chen SY, Menkari CE, Sulik KK, Charness ME., "Ethanol antagonist peptides: structural specificity without stereospecificity.", J Pharmacol Exp Ther. 2004 Jun, 309(3): 1183-9. Although the amino acids constituting the polypeptide may be partially a D-amino acid(s), it is preferred that the amino acids constituting the polypeptide be entirely, rather than partially, D-amino acids, from the viewpoint of preventing inhibition of the activity of the polypeptide as much as possible.

The polypeptide used as an active ingredient in the present invention can be easily prepared by a conventional method using, for example, a commercially available peptide synthesizer.

The agent of the present invention may be composed only of the polypeptide, or may be formulated by mixing as appropriate with one or more of additives such as pharmaceutically acceptable vehicles, stabilizers, preservatives, buffers, solubilizers, emulsifiers, diluents, and isotonic agents suitable for each administration mode. Formulation methods and additives which may be used are well known in the field of formulation of pharmaceuticals, and any of the methods and additives may be used.

The agent of the present invention is used by local administration to the skin lesion to be treated, or to the skin portion requiring prophylaxis. Examples of the method of local administration include intracutaneous administration using an injection solution or drops, and topical administration using an ointment, cream, lotion, patch, or the like. The dose is not limited, and appropriately selected depending on, for example, symptoms, age, body weight, and/or administration method. The dose for the subject animal may be usually about 0.01 µg to 1 g per day, for example, about 1 µg to 10 mg, in terms of the amount of the active ingredient. The above-described dose may be given to a subject once or dividedly in several times. Depending on the degree of amelioration of symptoms, the agent may be regularly administered for several days to several months, once or several times per day, or one day or several times per several days.

The subject to which the agent of the present invention is to be administered is a mammal, and examples of the mammal include human, dog, cat, rabbit, and hamster. The agent of the present invention can be especially preferably used for human.

The agent of the present invention may be used alone, or may be used in combination with another therapeutic or prophylactic agent for pruritic dermatoses. Standard therapeutic agents which have been conventionally used for pruritic dermatoses are steroid drugs and antihistamines. Since the agent of the present invention has an action mechanism different from those of these standard therapeutic agents, the agent of the present invention can also be used in combination with a steroid drug(s) and/or the like.

From the viewpoint of the action of the LL-37 peptide to promote expression and production of Sema3A, the therapeutic or prophylactic agent of the present invention can be regarded as an agent for promoting expression or production of Sema3a. That is, the present invention provides an agent for promoting expression of sema3A, comprising as an active ingredient a polypeptide of the following (a) or (b').
(a) A polypeptide with the amino acid sequence of SEQ ID NO:1.
(b') A polypeptide with the same amino acid sequence as the amino acid sequence of SEQ ID NO:1 except that one or several amino acids are substituted, deleted, inserted, and/or added, the polypeptide having an activity to promote expression of Sema3A.

Whether or not the polypeptide of (b') has an activity to promote expression of Sema3A can be investigated by, for example, adding the polypeptide to epidermis-derived cultured cells (e.g., normal human epidermal keratinocytes (NHEKs)), measuring the expression level of Sema3A in the cells, and then comparing the measured expression level with the expression level of Sema3A in cells to which the polypeptide was not added. Other preferred conditions and the like for the polypeptide of (b') are the same as those for the polypeptide of (b).

As mentioned above, it is known that application of an ointment containing a recombinant Sema3A to lesions of atopic dermatitis allows alleviation of pruritus and amelioration of the dermatitis (Non-patent Document 8). By the agent for promoting Sema 3A expression containing as an active ingredient the polypeptide of (a) or (b'), expression of Sema3A can be promoted, and the amount of production of Sema3A can be increased in epidermal keratinocytes, so that treatment or prevention of pruritic dermatoses such as atopic dermatitis becomes possible.

### EXAMPLES

The present invention is described below more concretely by way of Examples. However, the present invention is not limited to these Examples.

### 1. Effect of Treatment with Human Cathelicidin LL-37 on Expression of Nerve Repulsion Factors and the Like in Human Epidermal Keratinocytes

### [Materials and Methods]

### (1) Synthesis of LL-37

The LL-37 peptide (LLGDFFRKSKEKIGKEFKRIVQRIKDFLRNLVPRTES, SEQ ID NO:1) was synthesized using an automatic peptide synthesizer (manufactured by Shimadzu Corporation, model PSSM-8) according to a protocol in the manufacturer's instructions for the synthesizer.

### (2) Culture of NHEKs

Normal human epidermal keratinocytes (NHEKs) were cultured according to a protocol provided by Lonza. NHEKs were cultured in a 12-well tissue culture plate, and, in the growth phase (80% confluence), LL-37 (20 µg/ml) was added alone, or LL-37 (20 µg/ml) and the peptide SLIGRL (SEQ ID NO:2, 100 µM) were added, to the cells. The peptide SLIGRL is a peptide frequently used as a PAR2 agonist, and it is known that activation of PAR2 induces promotion of expression of TSLP in keratinocytes (Wilson et al, Cell, 2013: 155, 285-295). Total RNA was extracted 48 hours after the addition of LL-37, and mRNA expression analysis was carried out by real-time PCR. The culture supernatant was collected, and subjected to analysis of the protein expression level by ELISA.

### (3) Analysis of Expression of mRNA of Each Gene

The transcription level of each gene was analyzed by quantitative real-time PCR. Total RNA was extracted from the cultured cells using an RNeasy Mini Kit (QIAGEN) according to the manufacturer's instructions, and subjected to reverse transcription into cDNA using a PrimeScript RT Reagent Kit (Takara). According to the manufacturer's instructions, quantitative real-time PCR was carried out using an Applied Biosystems 7900HT Fast Real-time PCR system (Applied Biosystems) with SYBR Premix Ex Taq (Takara). Table 1 shows information on the primers used. A ribosome protein S18 (RPS18) was used as a reference gene. The amount of each mRNA was normalized against the amount of mRNA of RPS 18, and finally expressed as the ratio to the mRNA in an untreated control.

**[Table 1]**

| Gene | Base sequences of primers | SEQ ID NO |
|---|---|---|
| Sema3A | (forward) ACCCAACTATCAATGGGTGCCTTA | 3 |
| | (reverse) AACACTGGATTGTACATGGCTGGA | 4 |
| NGF | (forward) CATGCTGGACCCAAGCTCA | 5 |
| | (reverse) CCTGCAGGGACATTGCTCTC | 6 |
| KAL1 | (forward) CAACCGGATCAGAGGCATCA | 7 |
| | (reverse) TTGGCCGTATTGGTTGGACA | 8 |
| Artemin | (forward) CCCGGAAAGGTGCCTAGAAGA | 9 |
| | (reverse) ATCAGCCATTGTTGAGCTGTTCC | 10 |
| hBD-1 | (forward) TTGTCTGAGATGGCCTCAGGTGGTAAC | 11 |
| | (reverse) ATACTTCAAAAGCAATTTTCCTTTAT | 12 |
| hBD-2 | (forward) ATCAGCCATGAGGGTCTTGT | 13 |
| | (reverse) GAGACCACAGGTGCCAATTT | 14 |
| TSLP | (forward) TTTATACCTGTTGGCCCTGCCTA | 15 |
| | (reverse) CTTGCAGCGGGAATTCAAGA | 16 |
| RPS18 | (forward) TTTGCGAGTACTCAACACCAACATC | 17 |
| | (reverse) GAGCATATCTTCGGCCCACAC | 18 |

### (4) Analysis of Protein Expression

The expression level of each protein was analyzed by ELISA. The amount of Sema3A in the culture supernatant was measured using an Enzyme-linked Immunosorbent Assay Kit (USCN Life Science) according to the manufacturer's instructions.

### [Results]

After the addition of LL-37, increases in the expression of mRNAs of Sema3A, KAL1, hBD-1, and hBD-2, and decreases in the expression of mRNAs of NGF, Artemin, and TSLP were found in the normal human epidermal keratinocytes (NHEKs) (Fig. 1, Fig. 2, Fig. 3, and Fig. 4). The mRNA expression of TSLP also decreased by adding LL-37 to the NHEKs when the TSLP expression was induced in the NHEKs by adding thereto SLIGRL (Fig. 4).

An increase in the amount of Sema3A protein in the culture supernatant of the normal human epidermal keratinocytes (NHEKs) was also found after the addition of LL-37 (Fig. 5). From this result, it was suggested that the addition of LL-37 increased the mRNA expression of Sema3A in the normal human epidermal keratinocytes (NHEKs), resulting in production of Sema3A protein and its secretion into the culture supernatant.

Sema3A and KAL1 are known as nerve repulsion factors, which are molecules responsible for determining the direction of elongation of the nerve axon. In the presence of a nerve repulsion factor, elongation of the nerve axon is inhibited.

NGF and Artemin are known as nerve growth factors, which are molecules responsible for determining the direction of elongation of the nerve axon. In the presence of a nerve growth factor, elongation of the nerve axon is promoted.

TSLP is a cytokine belonging to the IL-2 family, and reported to be highly expressed in epidermal lesions of patients with atopic dermatitis (Soumelis et al, Nat Immunol, 2002: 3, 673-680).

From these results, it was suggested that LL-37 may normalize expression of nerve repulsion factors and nerve growth factors in lesions of atopic dermatitis. It was further suggested that LL-37 may induce expression of the antimicrobial peptides hBD-1 and hBD-2, and suppress expression of the cytokine TSLP. External application of an ointment containing LL-37 to lesions of atopic dermatitis may enable supplementation of the antimicrobial activity against *Staphylococcus aureus* exerted by LL-37 itself and hBD induced by LL-37, as well as normalization of expression of axon guidance molecules and TSLP, so that more effective amelioration of pathological conditions of atopic dermatitis can be expected.

### 2. Suppression of Scratching Behavior in Atopic Dermatitis Model Mouse Using Mouse Cathelicidin mCRAMP

### [Materials and Methods]

### (1) Preparation of Atopic Dermatitis Model Mouse

According to a protocol provided by Biostir Inc., Biostir AD (Biostir Inc.), which is an ointment reagent containing a *Dermatophagoides farinae* body (Dfb) component, was repeatedly applied to the back of NC/Nga mice (Oriental Yeast Co., Ltd.) by carrying out the application twice a week for 3 weeks, to induce atopic dermatitis. The clinical skin score was determined on the day before initiation of the application of the antimicrobial peptide mCRAMP ointment (Suto et al., Int Arch Allergy Immunol 120 (Suppl. 1), 70-75, 1999), and mice with scores of not less than 5 were used for the experiment.

### (2) Application of mCRAMP ointment to Atopic Dermatitis Model Mouse

To Dfb-NC/Nga mice which developed atopic dermatitis, mCRAMP ointment was applied twice a day for 4 days at 100 mg/mouse. The mCRAMP ointment was prepared by mixing 20 µl of an mCRAMP solution (10 µg/ml, ANASPEC Inc.) with 100 mg of hydrophilic vaseline (Maruishi Pharmaceutical Co., Ltd.). To the mice in the control group (vaseline application), 20 µl of distilled water mixed with 100 mg of hydrophilic vaseline was applied. mCRAMP (GLLRKGGEKIGEKLKKIGQKIKNFFQKLVPQPEQ, SEQ ID NO:19) is a mouse cathelicidin-related antimicrobial peptide, and corresponds to human LL-37.

### (3) Analysis of Scratching Behavior

Using SCLABA (registered trademark)-Real (Noveltec Inc.), measurement and analysis of scratching behavior was carried out. After the development of atopic dermatitis, each mouse was subjected to 12-hour measurement of scratching behavior before the application of the mCRAMP ointment (Day 0) and after the application of the mCRAMP ointment (Day 4).

### [Results]

To investigate the therapeutic effect of mCRAMP ointment application on the atopic dermatitis model mice, the ratio of change in the scratching behavior was compared between mice which developed atopic dermatitis but did not yet receive ointment application (Day 0) and mice which received ointment application (Day 4). As a result, the scratching behavior of the atopic dermatitis model mice was apparently suppressed by application of the mCRAMP ointment (Fig. 6).

## Claims

1. A therapeutic or prophylactic agent for pruritic dermatosis comprising as an active ingredient a polypeptide of the following (a) or (b):
(a) a polypeptide with the amino acid sequence of SEQ ID NO:1;
(b) a polypeptide with the same amino acid sequence as the amino acid sequence of SEQ ID NO:1 except that one or several amino acids are substituted, deleted, inserted, and/or added, said polypeptide having therapeutic and/or prophylactic activity for pruritic dermatosis.

2. The therapeutic or prophylactic agent according to claim 1, wherein said polypeptide of (b) has a sequence identity of not less than 80% to the amino acid sequence of SEQ ID NO:1.

3. The therapeutic or prophylactic agent according to claim 2, wherein said polypeptide of (b) has a sequence identity of not less than 90% to the amino acid sequence of SEQ ID NO:1.

4. The therapeutic or prophylactic agent according to claim 1, comprising as an active ingredient a polypeptide with the amino acid sequence of SEQ ID NO:1.

5. The therapeutic or prophylactic agent according to any one of claims 1 to 4, wherein said pruritic dermatosis is atopic dermatitis.

6. An agent for promoting expression of semaphorin 3A, comprising as an active ingredient a polypeptide of the following (a) or (b'):
(a) a polypeptide with the amino acid sequence of SEQ ID NO:1;
(b') a polypeptide with the same amino acid sequence as the amino acid sequence of SEQ ID NO:1 except that one or several amino acids are substituted, deleted, inserted, and/or added, said polypeptide having an activity to promote expression of semaphorin 3A.

7. The agent according to claim 6, which is a therapeutic or prophylactic agent for pruritic dermatosis.

8. A method of treating or preventing pruritic dermatosis, said method comprising administering an effective amount of a polypeptide of the following (a) or (b) to a subject in need thereof:
(a) a polypeptide with the amino acid sequence of SEQ ID NO:1;
(b) a polypeptide with the same amino acid sequence as the amino acid sequence of SEQ ID NO:1 except that one or several amino acids are substituted, deleted, inserted, and/or added, said polypeptide having therapeutic and/or prophylactic activity for pruritic dermatosis.

9. A method of promoting expression of semaphorin 3A, said method comprising administering an effective amount of a polypeptide of the following (a) or (b) to a subject in need thereof:
(a) a polypeptide with the amino acid sequence of SEQ ID NO:1;
(b') a polypeptide with the same amino acid sequence as the amino acid sequence of SEQ ID NO:1 except that one or several amino acids are substituted, deleted, inserted, and/or added, said polypeptide having an activity to promote expression of semaphorin 3A.
